# EUROPEAN PATENT APPLICATION

(11) **EP 4 534 168 A1**
(43) Date of publication of application: **09.04.2025**
(21) Application number: 23203267.2
(22) Date of filing: 12.10.2023
(51) Int. Cl.: B01D 11/04, B01D 3/40, C07C 7/08, C07C 7/10, C10G 21/12

(54) **A PROCESS FOR SEPARATING C6-C9-AROMATIC HYDROCARBONS AND ISOPRENE USING AN IMPROVED EXTRACTING AGENT**

(30) Priority: 06.10.2023 US 202363542971 P
(71) Applicant: Sulzer Management AG, 8401 Winterthur (CH)
(72) Inventor: The designation of the inventor has not yet been filed
(74) Representative: Henkel & Partner mbB

(57) **Abstract**

The present invention relates to a process for separating one or more compounds being selected from the group consisting of C₆-C₉-aromatic hydrocarbons, isoprene and arbitrary mixtures thereof from a feed composition including the one or more compounds in admixture with at least one other organic and/or inorganic compound by liquid-liquid extraction or by extractive distillation using an extracting agent, wherein the extracting agent comprises i) sulfolane and ii) at least one cosolvent selected from the group consisting of 3-methyl sulfolane and a sulfone according to the general formula (I) R₁R₂SO₂, wherein R₁ and R₂ are independently from each other a C₁-C₄-alkyl radical.

## Description

The present invention relates to a process for separating C₆-C₉-aromatic hydrocarbons and isoprene thereof from a feed composition including the one or more compounds in admixture with at least one other organic and/or inorganic compound.

Separating one or more specific compounds from a mixture containing the one or more specific compounds in admixture with other chemical compounds is an important task in chemical processes. A plurality of separation methods is available, such as distillation, extraction, absorption, adsorption, ion exchange chromatography and many others. A very common separation technique used to separate a mixture of several organic compounds is distillation, which allows to separate volatile organic compounds based on their different boiling points. During the distillation, the mixture to be separated is heated, which causes that compounds with lower boiling points vaporize and rise to the top of the distillation column, where they are withdrawn as overhead stream, condensed and are then further processed according to the need. In contrast thereto, compounds with higher boiling points do not vaporize, but remain as liquid and flow down the distillation column to the bottom of the distillation column, where they are removed as bottom stream and are then further processed according to the need. However, distillation is not applicable to separate different chemical compounds having a similar boiling point.

For separating such mixtures comprising different chemical compounds having a similar or even identical boiling point, liquid-liquid extraction and extractive distillation are promising alternatives. Extraction uses an extracting agent, in which the chemical compound to be separated from a mixture has a drastic higher solubility than the other chemical compound(s) of the mixture from which the chemical compound shall be separated. During the operation, the mixture and extracting agent are led, co-currently or counter-currently, through an extraction column, in which both streams are contacted with each other. On account of its higher solubility in the extracting agent, most or ideally virtually all of the chemical compound to be separated from the mixture is dissolved in the extracting agent, thus forming an extraction composition, which is withdrawn from the extraction column separately from the remaining raffinate, which contains the other chemical compound(s). In turn, extractive distillation is a distillation, in which an extracting agent is fed in addition to the mixture into the distillation column, wherein the extracting agent is so selected that it increases the relative volatility of the chemical compounds to be separated. Relative volatility is an indication of the degree of the separability or ease of separation, respectively, of two or more chemical compounds by distillation from a mixture of these chemical compounds. An additional requirement for the extracting agent being used in extractive distillation is that it has a significant different boiling point than the chemical compounds to be separated.

Sulfolane and mixtures of sulfolane and water are commonly used to separate C₆-C₉-aromatic hydrocarbons, such as benzene, styrene or the like, from nonaromatic compounds having a very similar or even essentially identical boiling point. Even if sulfolane is a quite suitable extracting agent, it is, even at a comparable high level, limited concerning the selectivity and solvency with regard to C₆-C₉-aromatic hydrocarbons and isoprene to be separated from other chemical compounds having a highly similar boiling point. Therefore, using sulfolane or a mixture of sulfolane and water, a relatively high amount of energy is required to separate a predetermined volume of the mixture, which avoids a higher profitability of the separation process. Furthermore, it is nowadays desired to reduce the energy demand for processes as much as possible for environmental reason and to reduce thereby carbon emissions being connected with the energy production.

In view of this, the object underlying the present invention is to provide a process for separating one or more compounds being selected from the group consisting of C₆-C₉-aromatic hydrocarbons, isoprene and arbitrary mixtures thereof from a feed composition including the one or more compounds in admixture with at least one other organic and/or inorganic compound by liquid-liquid extraction or by extractive distillation making use of an extracting agent having an increased selectivity and improved solvency with regard to C₆-C₉-aromatic hydrocarbons and isoprene so that the process is characterized by a reduced energy demand, per unit of separated mixture, and thus by reduced carbon emissions being connected with the energy production and with an increased profitability of the process.

In accordance with the present invention, this object is satisfied by providing a process for separating one or more compounds being selected from the group consisting of C₆-C₉-aromatic hydrocarbons, isoprene and arbitrary mixtures thereof from a feed composition including the one or more compounds in admixture with at least one other organic and/or inorganic compound by liquid-liquid extraction or by extractive distillation using an extracting agent, wherein the extracting agent comprises i) sulfolane and ii) at least one co-solvent selected from the group consisting of 3-methyl sulfolane and a sulfone according to the general formula (I) R₁R₂SO₂, wherein R₁ and R₂ are independently from each other a C₁-C₄-alkyl radical.

This solution bases on the surprising finding that a mixture comprising i) sulfolane and ii) at least one co-solvent selected from the group consisting of 3-methyl sulfolane and a sulfone according to the general formula (I) R₁R₂SO₂, wherein R₁ and R₂ are independently from each other a C₁-C₄-alkyl radical, has a higher selectivity and solvency with regard to C₆-C₉-aromatic hydrocarbons and isoprene to be separated from other chemical compounds having a highly similar boiling point than sulfolane alone or than a mixture of sulfolane and water.

The present invention is not particularly limited concerning the contents and ratios of sulfolane and the co-solvent. However, in tendency, the higher the total amount of both components i) and ii), the better the performance of the extracting agent. Good results are in particular obtained, when the extracting agent comprises, based on 100% by weight of the extracting agent, at least 80% by weight, more preferably at least 90% by weight and still more preferably at least 95% by weight of sum i) sulfolane and ii) at least one co-solvent selected from the group consisting of 3-methyl sulfolane and a sulfone according to the general formula (I).

In accordance with a preferred embodiment of the present invention, the extracting agent consists or at least essentially consists of a mixture of i) sulfolane and ii) at least one co-solvent selected from the group consisting of 3-methyl sulfolane and a sulfone according to the general formula (I), i.e. the total amount of the components i) and ii) is, based on 100% by weight of the extracting agent, at least 98% by weight, preferably at least 99% by weight, still more preferably at least 99.5% by weight and most preferably 100% by weight.

In accordance with an alternative preferred embodiment of the present invention, the extracting agent contains high amounts of the components i) and ii) and in addition thereto a comparable small amount of water. It has been surprisingly found by the inventors of the present invention that the addition of a comparable small amount of water to a mixture if i) sulfolane and ii) at least one co-solvent selected from the group consisting of 3-methyl sulfolane and a sulfone according to the general formula (I) even further increases the selectivity and solvency with regard to C₆-C₉-aromatic hydrocarbons and isoprene in comparison to other chemical compounds having a highly similar boiling point. It is preferred in this embodiment that the extracting agent comprises, based on 100% by weight of the extracting agent, a) 90 to 100% by weight, preferably 95 to 99% by weight and more preferably 97 to 99% by weight of sum of i) sulfolane and of ii) at least one co-solvent selected from the group consisting of 3-methyl sulfolane and a sulfone according to the general formula (I) and b) 1 to 10% by weight, preferably 1 to 5% by weight and more preferably 1 to 3% by weight of water.

As set out above, the present invention is not particularly limited concerning the ratios of sulfolane and the co-solvent, i.e. regarding the relative amounts of sulfolane and the co-solvent, in the extracting agent. Good results are in particular obtained, when the extracting agent contains about equal amounts of the components i) and ii) or a higher amount of component i) than of component ii). Accordingly, it is preferred that the extracting agent comprises, based on 100% by weight of the extracting agent, 40 to 90% by weight of i) sulfolane and 10 to 60% by weight of ii) at least one co-solvent selected from the group consisting of 3-methyl sulfolane and a sulfone according to the general formula (I). Particular good results are obtained, when the extracting agent comprises, based on 100% by weight of the extracting agent, more than 50 to 90% by weight of i) sulfolane and 10 to less than 50% by weight of ii) at least one co-solvent selected from the group consisting of 3-methyl sulfolane and a sulfone according to the general formula (I), such as 60 to 75% by weight of i) sulfolane and 25 to less than 40% by weight of ii) at least one co-solvent selected from the group consisting of 3-methyl sulfolane and a sulfone according to the general formula (I).

In accordance with the present invention, the extracting agent comprises i) sulfolane and ii) at least one co-solvent selected from the group consisting of 3-methyl sulfolane and a sulfone according to the general formula (I). Good results are in particular obtained, when the extracting agent comprises i) sulfolane and ii) (only) one co-solvent selected from the group consisting of 3 methyl sulfolane and a sulfone according to the general formula (I) R₁ R₂SO₂, but no mixture thereof

In accordance with the present invention, the sulfone according to the general formula (I) R₁R₂SO₂, in which R₁ and R₂ are independently from each other a C₁-C₄-alkyl radical.

In accordance with a preferred embodiment of the present invention, the radicals R₁ and R₂ in the general formula (I) are the same. Accordingly, it is in this embodiment preferred that the extracting agent comprises as co-solvent ii) dimethylsulfone, diethylsulfone, dipropylsulfone or dibutylsulfone.

In accordance with an alternatively preferred embodiment of the present invention, the radicals R₁ and R₂ in the general formula (I) are the different to each other. Suitable examples for co-solvents of this embodiment are methyl ethyl sulfone, methyl propyl sulfone, methyl butyl sulfone, ethyl propyl sulfone and ethyl butyl sulfone. More preferably, in this embodiment one of the radicals R₁ and R₂ in the general formula (I) is hydrogen, whereas the other radical is a C₁-C₄-alkyl radical. Examples for the co-solvent of this variant are methylsulfone, ethylsulfone, propylsulfone and butylsulfone, wherein more preferred examples are n-propylsulfone, iso-propylsulfone, n-butylsulfone and iso-butylsulfone.

In accordance with a particular preferred embodiment of the present invention, the extracting agent comprises as co-solvent ii) 3-methyl sulfolane, dimethylsulfone, diethylsulfone or n-propylsulfone. Still more preferably the extracting agent comprises as co-solvent ii) 3-methyl sulfolane, dimethylsulfone or n-propylsulfone and most preferably the extracting agent comprises as co-solvent ii) dimethylsulfone.

In view of the above, it is particularly preferred that the extracting agent comprises, based on 100% by weight of the extracting agent, more than 50 to 90% by weight of i) sulfolane, 10 to less than 50% by weight of ii) at least one co-solvent and 1 to 3% by weight of water, wherein the co-solvent ii) is 3-methyl sulfolane, dimethylsulfone, diethylsulfone or n-propylsulfone and more preferably dimethylsulfone.

The present invention is not particularly restricted concerning the kind of feed composition, as long as it contains one or more compounds being selected from the group consisting of C₆-C₉-aromatic hydrocarbons, isoprene and arbitrary mixtures thereof in admixture with at least one other organic and/or inorganic compound. The process in accordance with the present invention is in particular suitable for a feed composition, which comprises, based on 100% by weight of the feed composition, 10 and 90% by weight and preferably 60 and 90 % by weight of C₆-C₉-aromatic hydrocarbons and/or isoprene and 10 to 90% by weight and preferably 10 and 40 % by weight of at least one other organic compound.

In a further development of the idea of the present invention, it is suggested that the feed composition contains one or more C₆-C₉-aromatic hydrocarbons being selected from the group consisting of benzene, toluene, xylenes, ethylbenzene, styrene and arbitrary mixtures thereof.

The at least one other organic compound may be for instance selected from the group consisting of paraffins, cycloalkanes, olefins, cycloolefins and arbitrary mixtures thereof. Good results are in particular obtained, when the feed composition contains at least one other organic compound being selected from the group consisting of normal paraffins, iso-paraffins, cycloalkanes, normal olefins, iso-olefins and C₄-C₁₀-cycloolefins.

For instance, the feed composition may be a pyrolysis gasoline, a reformate, an oil mixture obtained from a plastic recycle process or any other organic chemical stream being rich in aromatics and in particular being rich in C₆-C₉-aromatics. Most preferably, the feed composition is a pyrolysis gasoline or a reformate.

In accordance with the present invention, one or more compounds being selected from the group consisting of C₆-C₉-aromatic hydrocarbons, isoprene and arbitrary mixtures thereof are separated from the feed composition by liquid-liquid extraction or by extractive distillation using the aforementioned extracting agent. Concerning the kind of liquid-liquid extraction or of the extractive distillation, the present invention is not particularly limited.

The present invention is not particularly limited concerning the kind of liquid-liquid-extraction column used for the process. Thus, all conventional liquid-liquid-extraction columns may be used. Good results are in particular obtained, when the one or more compounds being selected from the group consisting of C₆-C₉-aromatic hydrocarbons, isoprene and arbitrary mixtures thereof are separated from the feed composition by liquid-liquid extraction using the extracting agent, wherein the liquid-liquid extraction is performed in a spray extraction column, in a pulsed extraction column, such as in a packed extraction column or in a in a sieve tray extraction column, or in an agitated extraction column, such as in an agitated counter-current extraction column. For instance, the agitated extraction column comprises one or more rotating shafts, each of which comprising one or more agitators being preferably selected from the group consisting of discs, blades, paddles, turbine impellers, fins and arbitrary combinations of two or more of the aforementioned agitators.

In an alternatively preferred embodiment of the present invention, the one or more compounds being selected from the group consisting of C₆-C₉-aromatic hydrocarbons, isoprene and arbitrary mixtures thereof are separated from the feed composition by extractive distillation using the extracting agent in a distillation column, wherein the distillation column comprises an inlet for the feed composition, an inlet for the feed composition, an overhead outlet and a bottom outlet.

Subsequently, the present invention is illustrated by means of non-limiting examples and figures.

In the figures:
- Figure 1: shows the relative volatility for the vapor-liquid-equilibrium of a mixture containing n-octane and toluene using as solvent a mixture of 3-methyl sulfolane and sulfolane (solid line) compared to the use of sul-folane alone (dotted line).
- Figure 2: shows the selectivity in liquid-liquid extraction for a mixture containing n-heptane and toluene using as solvent a mixture of dimethylsulfone and sulfolane (solid line) compared to the use of sulfolane alone (dot-ted line).

### Examples

### Example 1 and Comparative Example 1

As example 1, in a vapor-liquid-equilibrium (VLE) study experiment 75 g of a hydrocarbon mixture containing a mixture of 25% by weight of toluene and 75% by weight of n-octane was added together with a solvent mixture containing 20% by weight of 3-methyl sulfolane and 80% by weight of sulfolane into a VLE equilibrium cell. Three experiments were performed under three different solvent-to-feed ratios in weight/weight, namely with respective ratios of 3:1, of 5:1 and of 10:1 keeping the ratio between 3-methyl sulfolane and sulfolane in the solvent mixture constant and also keeping the amount of hydrocarbon mixture added to the VLE equilibrium cell constant. The entire mixture for each experiment was brought at boiling condition under constant pressure and once the equilibrium was reached, samples from vapor and liquid phase were collected and analyzed by gel chromatography (GC).

The same experiments as described above were conducted as comparative example 1 using only sulfolane as solvent.

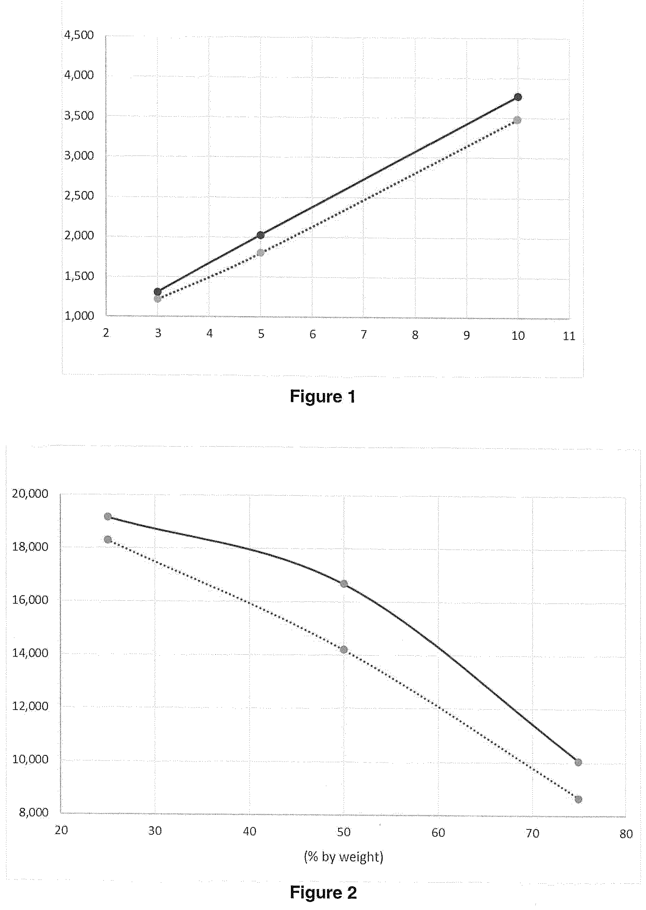

For all three experiments, the relative volatilities were calculated using classical formula for different aromatics/non-aromatics pairs. The plot of figure 1 shows how the relative volatility (shown on the ordinate) for the n-octane/toluene pair varies for the different solvent-to-feed ratios (shown on the abscissa) for example 1 (solid line) as well as for comparative example 1 (dotted line). When using a new solvent agent in an extractive distillation, the main purpose is to improve the relative volatility of the components as these leads automatically to an improved extractive distillation industrial process.

It can be derived from figure 1 that the relative volatilities of n-octane and toluene obtained in example 1 using a mixture of sulfolane and 3-methyl sulfolane as solvent mixture is higher and thus improved compared to those obtained in comparative example 1 using a sulfolane alsone as solvent.

### Example 2 and Comparative Example 2

As example 2, in a liquid-liquid extraction (LLE) study experiment 50 g of a hydrocarbon mixture containing n-heptane and toluene was added together with a solvent mixture containing 30% by weight of dimethylsulfone and 70% by weight of sulfolane in a LLE equilibrium cell. Three experiments were performed under three different aromatic contents, namely of 25% by weight, of 50% by weight and of 75% by weight keeping the ratio between dimethylsulfone and sulfolane constant and also keeping the amount of hydrocarbons added to the LLE equilibrium cell constant. For all three experiments the same solvent-to-feed ratio of 3:1 was used. The entire mixture for each experiment was rigorously mixed at constant temperature and once the equilibrium was reached, two liquid phases were separated (one being rich in non-aromatics with less solvent called "raffinate" and the other one being rich in aromatics and solvent called "extract"). Samples from both raffinate and extract phases were collected and analyzed by GC.

The same experiments as described above were conducted as comparative example 2 using only sulfolane as solvent.

For all three experiments, the selectivities were calculated using classical formula for different aromatics/non-aromatics pairs. The plot of figure 2 shows how the selectivity ty (shown on the ordinate) for the n-heptane/toluene pair varies for the different aromatic contents (shown on the abscissa) for example 1 (solid line) as well as for comparative example 1 (dotted line). When use a new solvent agent in liquid-liquid extraction, the main purpose is to improve the selectivity as this leads automatically to an improved liquid-liquid extraction industrial process.

It can be derived from figure 2 that the selectivity is improved, when the liquid-liquid extraction is conducted with a mixture of sulfolane and dimethylsulfone compared to using sulfolane alone as extracting agent.

## Claims

1. A process for separating one or more compounds being selected from the group consisting of C₆-C₉-aromatic hydrocarbons, isoprene and arbitrary mixtures thereof from a feed composition including the one or more compounds in admixture with at least one other organic and/or inorganic compound by liquid-liquid extraction or by extractive distillation using an extracting agent, wherein the extracting agent comprises i) sulfolane and ii) at least one co-solvent selected from the group consisting of 3-methyl sulfolane and a sulfone according to the general formula (I) R₁R₂SO₂, wherein R₁ and R₂ are independently from each other a C₁-C₄-alkyl radical.

2. The process in accordance with claim 1, wherein the total amount of i) sulfolane and ii) the at least one co-solvent selected from the group consisting of 3-methyl sulfolane and a sulfone according to the general formula (I) in the extracting agent is, based on 100% by weight of the extracting agent, at least 98% by weight, preferably at least 99% by weight, still more preferably at least 99.5% by weight and most preferably 100% by weight.

3. The process in accordance with claim 1, wherein the extracting agent comprises, based on 100% by weight of the extracting agent, a) 90 to 99% by weight, preferably 95 to 99% by weight and more preferably 97 to 99% by weight of sum of i) sulfolane and of ii) at least one co-solvent selected from the group consisting of 3-methyl sulfolane and a sulfone according to the general formula (I) and b) 1 to 10% by weight, preferably 1 to 5% by weight and more preferably 1 to 3% by weight of water.

4. The process in accordance with any of the preceding claims, wherein the extracting agent comprises, based on 100% by weight of the extracting agent, 40 to 90% by weight and preferably more than 50 to 90% by weight of i) sulfolane and 10 to 60% by weight and preferably 10 to less than 50% by weight of ii) at least one co-solvent selected from the group consisting of 3-methyl sulfolane and a sulfone according to the general formula (I).

5. The process in accordance with any of the preceding claims, wherein the extracting agent comprises i) sulfolane and ii) one co-solvent selected from the group consisting of 3 methyl sulfolane and a sulfone according to the general formula (I) R₁R₂SO₂.

6. The process in accordance with any of the preceding claims, wherein the extracting agent comprises as co-solvent ii) dimethylsulfone, diethylsulfone, dipropylsulfone or dibutylsulfone.

7. The process in accordance with any of the preceding claims, wherein the extracting agent comprises as co-solvent ii) n-propylsulfone, iso-propylsulfone, n-butylsulfone or iso-butylsulfone.

8. The process in accordance with any of the preceding claims, wherein the extracting agent comprises as co-solvent ii) 3-methyl sulfolane, dimethylsulfone, diethylsulfone or n-propylsulfone, preferably 3-methyl sulfolane, dimethylsulfone or n-propylsulfone and more preferably dimethylsulfone.

9. The process in accordance with any of the preceding claims, wherein the extracting agent comprises, based on 100% by weight of the extracting agent, more than 50 to 90% by weight of i) sulfolane, 10 to less than 50% by weight of ii) at least one co-solvent and 1 to 3% by weight of water, wherein the co-solvent ii) is 3-methyl sulfolane, dimethylsulfone, diethylsulfone or n-propylsulfone and preferably dimethylsulfone.

10. The process in accordance with any of the preceding claims, wherein the feed composition comprises, based on 100% by weight of the feed composition, 10 and 90% by weight and preferably 60 and 90 % by weight of C₆-C₉-aromatic hydrocarbons and/or isoprene and 10 to 90% by weight and preferably 10 and 40 % by weight of at least one other organic compound.

11. The process in accordance with any of the preceding claims, wherein the C₆-C₉-aromatic hydrocarbons are selected from the group consisting of benzene, toluene, xylenes, ethylbenzene, styrene and arbitrary mixtures thereof.

12. The process in accordance with claim 11, wherein the at least one other organic compound is selected from the group consisting of paraffins, cycloalkanes, olefins, cycloolefins and arbitrary mixtures thereof.

13. The process in accordance with any of the preceding claims, wherein the feed composition is a pyrolysis gasoline, a reformate, an oil mixture obtained from a plastic recycle process or any other organic chemical stream being rich in C₆-C₉-aromatics and preferably a pyrolysis gasoline or a reformate.

14. The process in accordance with any of the preceding claims, wherein the one or more compounds being selected from the group consisting of C₆-C₉-aromatic hydrocarbons, isoprene and arbitrary mixtures thereof are separated from the feed composition by liquid-liquid extraction using the extracting agent, wherein the liquid-liquid extraction is performed in a spray extraction column, in a pulsed extraction column or in an agitated extraction column and preferably in an agitated counter-current extraction column, in a packed extraction column or in a in a sieve tray extraction column.

15. The process in accordance with any of the preceding claims, wherein the one or more compounds being selected from the group consisting of C₆-C₉-aromatic hydrocarbons, isoprene and arbitrary mixtures thereof are separated from the feed composition by extractive distillation using the extracting agent in a distillation column, wherein the distillation column comprises an inlet for the feed composition, an inlet for the feed composition, an overhead outlet and a bottom outlet.
